# EUROPEAN PATENT SPECIFICATION

(11) **EP 3 124 599 B1**
(45) Date of publication and mention of the grant of the patent: **24.07.2019**
(21) Application number: 15768699.9
(22) Date of filing: 23.03.2015
(51) Int. Cl.: A61K 35/74, A61K 31/7004, C12N 1/20, C12R 1/01, A61P 1/02, A23L 33/135

(54) **LACTIC ACID BACTERIUM BELONGING TO GENUS WEISSELLA**
ZUR GATTUNG WEISSELLA GEHÖRENDES MILCHSÄUREBAKTERIUM
BACTÉRIE LACTIQUE APPARTENANT AU GENRE WEISSELLA

(30) Priority: 28.03.2014 JP 2014069383
(43) Date of publication of application: 01.02.2017
(73) Proprietor: Lotte Co., Ltd., Tokyo 160-0023 (JP)
(72) Inventor: DOI, Teppei, Saitama-shi Saitama 336-8601 (JP); SAEKI, Yoji, Saitama-shi Saitama 336-8601 (JP)
(74) Representative: Blodig, Wolfgang
(86) International application number: PCT/JP2015/001629
(87) International publication number: WO 2015/146133

(56) References cited:
- JP-A- H03 176 418
- JP-A- 2007 320 926
- KR-A- 20040 092 931
- KR-B1- 100 995 357
- CHOI HAK-JONG ET AL: "Weissella kimchii sp. nov., a novel lactic acid bacterium from kimchi.", INTERNATIONAL JOURNAL OF SYSTEMATIC AND EVOLUTIONARY MICROBIOLOGY MAR 2002, vol. 52, no. Pt 2, March 2002 (2002-03), pages 507-511, XP002774090, ISSN: 1466-5026
- KANG, M. S. ET AL.: 'Effect of Weissella cibaria Isolates on the Formation of Streptococcus mutans Biofilm' CARIES RESEARCH vol. 40, 2006, pages 418 - 425, XP008184397
- LEE, Y.: 'Promotion of Bone Nodule Formation and Inhibition of Growth and Invasion of Streptococcus mutans by Weissella kimchii PL 9001' JOURNAL OF MICROBIOLOGY AND BIOTECHNOLOGY vol. 16, no. 4, 2006, pages 531 - 537, XP055358502

### SEQUENCE LISTING

<110> LOTTE CO., LTD
<120> Weissella bacterium
<130> LOTTE-163WO
<150> JP 2014-069383
   <151> 2014-03-28
<160> 3
<170> PatentIn version 3.5
<210> 1
   <211> 20
   <212> DNA
   <213> Artificial Sequence
<220>
   <223> Primer
<400> 1
   agagtttgat cctggctcag 20
<210> 2
   <211> 16
   <212> DNA
   <213> Artificial Sequence
<220>
   <223> Primer
<400> 2
   attaccgcsg ctgctg 16
<210> 3
   <211> 483
   <212> DNA
   <213> Unknown
<220>
   <223> Weissella w3
<400> 3
   ctttgtggtt caactgattt gaagagcttg ctcagatatg acgatggaca ttgcaaagag      60
   tggcgaacgg gtgagtaaca cgtgggaaac ctacctctta gcaggggata acatttggaa     120
   acagatgcta ataccgtata acaatgacaa ccgcatggtt gttatttaaa agatggttct     180
   gctatcacta agagatggtc ccgcggtgca ttagctagtt ggtaaggtaa tggcttacca     240
   aggcgatgat gcatagccga gttgagagac tgatcggcca caatgggact gagacacggc     300
   ccatactcct acgggaggca gcagtaggga atcttccaca atgggcgaaa gcctgatgga     360
   gcaacgccgc gtgtgtgatg aagggtttcg gctcgtaaaa cactgttgta agagaagaat     420
   gacattgaga gtaactgttc aatgtgtgac ggtatcttac cagaaaggaa cggctaaata     480
   cgt                                                                   483

## Description

### Technical Field

The present invention relates to utilization of a microorganism for promoting health.

### Background Art

Lactic acid bacteria (e.g. *Lactobacillus* and *Bifidobacterium*) that contribute to the improved balance of the intestinal flora are known as microorganisms that provide beneficial effects to hosts. It has been sought to intake the microorganisms through food products and the like.

Meanwhile, utilization of microorganisms for promoting health not only in the intestine but also in the oral cavity has been recently attracting attention. For example, it has been reported in Japan that yogurt containing a lactic acid bacterium, *Lactobacillus* reuteri, inhibits growth of mutans streptococci which are bacteria contributing to dental decay and an example thereof has been reported in which the yogurt actually reduced the number of mutans streptococci in saliva in the tests on human subjects (NPL 1).

### Citation List

### Patent Literature

PTL 1: Korean Patent No. 10-0995357

### Non Patent Literature

NPL 1: Int. J. Food Microbiol. 95, pp. 219-23 (2004)
NPL 2: Journal of Intestinal Microbiology 21, pp. 129-142 (2007)
NPL 3: Caries Res. 40, pp. 418-25 (2006)
NPL 4: Int. J. Syst. Evol. Microbiol., 52, pp. 141-8 (2002)

### Summary of Invention

### Technical Problem

A problem to be solved by the present invention is to provide a lactic acid bacterium effective for promoting health, particularly for prophylaxis or therapy of oral diseases.

### Solution to problem

The inventors of the present application searched for lactic acid bacteria that are effective for promoting health, particularly for prophylaxis or therapy of oral diseases, and found that a lactic acid bacterium belonging to the genus *Weissella,* namely a lactic acid bacterium W3 belonging to the genus *Weissella* (*Weissella sp.* (w3): accession number: NITE BP-01789) is effective for prophylaxis or therapy of oral diseases.

### Advantageous Effects of Invention

A specific lactic acid bacterium belonging to the genus *Weissella,* namely the lactic acid bacterium W3 belonging to the genus *Weissella* suppresses biofilm formation by *S. mutans* and is effective for prophylaxis or therapy of oral diseases. Moreover, the specific lactic acid bacterium belonging to the genus *Weissella* is effective for prophylaxis or therapy of oral diseases when the bacterium is combined with xylose. *S. mutans* less likely metabolises xylose, and thus xylose is effective for prophylaxis or therapy of oral diseases when xylose is combined with a specific lactic acid bacterium belonging to the genus *Weissella,* namely the lactic acid bacterium W3 belonging to the genus *Weissella.*

### Brief Description of Drawings

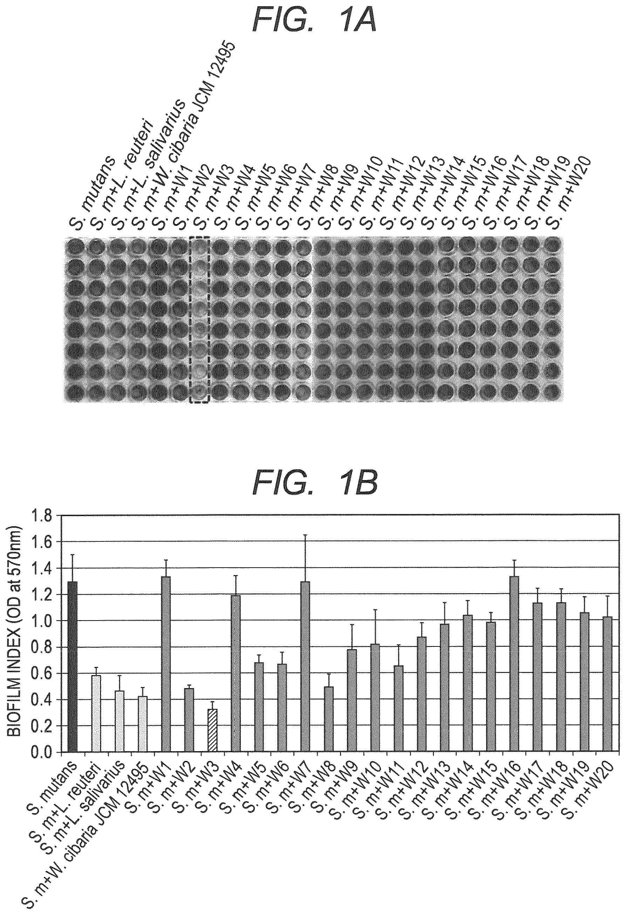

Fig. 1A shows the results of the experiment for examining the *S. mutans* biofilm formation suppressing effect by strains W1 to W20 and control strains and shows the intensity of colour of the observed biofilms stained in the experiment. The experiment shown in this figure was carried out with n = 8. Fig. 1B shows the results of the experiment for examining the *S. mutans* biofilm formation suppressing effect by strains W1 to W20 and control strains and the results are indicated as the absorbance obtained by numerically converting the results shown in Fig. 1A. The experiment shown in this figure was carried out with n = 8.
Fig. 2 shows pH at which *S. mutans* metabolises various carbohydrates.
Fig. 3 shows the growth curves of the lactic acid bacterium W3 belonging to the genus *Weissella.*
Fig. 4A shows the influence of the sucrose concentration and the influence of xylose on the *S.*
*mutans* biofilm formation suppressing effect by the lactic acid bacterium W3 belonging to the genus *Weissella* and shows the intensity of colour of the observed biofilms stained in the experiment.
Fig. 4B shows the influence of the sucrose concentration and the influence of xylose on the *S.*
*mutans* biofilm formation suppressing effect by the lactic acid bacterium W3 belonging to the genus *Weissella* and the results are indicated as the absorbance obtained by numerically converting the results shown in Fig. 4A.
Fig. 5A shows the xylose concentration dependency of the *S. mutans* biofilm formation suppressing effect by the lactic acid bacterium W3 belonging to the genus *Weissella* and shows the intensity of colour of the observed biofilms stained in the experiment. The experiment shown in this figure was carried out with n = 8.
Fig. 5B shows the xylose concentration dependency of the *S. mutans* biofilm formation suppressing effect by the lactic acid bacterium W3 belonging to the genus *Weissella* and the results are indicated as the absorbance obtained by numerically converting the results shown in Fig. 5A. The experiment shown in this figure was carried out with n = 8.
Fig. 6 is a micrograph of the Gram-stained lactic acid bacterium W3 belonging to the genus *Weissella.*

### Description of Embodiments

The present invention provides a lactic acid bacterium W3 belonging to the genus *Weissella (Weissella sp.* (w3): accession number: NITE BP-01789) or a mutant or transformant thereof. The lactic acid bacterium W3 belonging to the genus *Weissella* may be herein referred to as *"Weissella* lactic acid bacteria W3", *"Weissella* W3" or merely "W3".

A mutant refers to a bacterium having a slightly modified nature, phenotype, gene sequence or the like after succession of culture, and a mutated lactic acid bacterium W3 belonging to the genus *Weissella* is encompassed by the present invention as far as the bacterium is effective for prophylaxis or therapy of oral diseases, which is the problem of the present invention. A transformant refers to the lactic acid bacterium of the present invention having a gene artificially modified by means of a plasmid, a virus or other genetic engineering methods for industrial or medical purpose, and a transformed lactic acid bacterium W3 belonging to the genus *Weissella* is encompassed by the present invention as far as the bacterium is effective for prophylaxis or therapy of oral diseases, which is the problem of the present invention.

The present invention also provides an oral composition for prophylaxis or therapy of an oral disease comprising a lactic acid bacterium belonging to the genus *Weissella* and xylose. In the oral composition, the lactic acid bacterium belonging to the genus *Weissella* is particularly preferably a lactic acid bacterium W3 belonging to the genus *Weissella.*

The present invention also provides a food or beverage comprising a lactic acid bacterium W3 strain belonging to the genus *Weissella* or a mutant thereof or transformant thereof or the above oral composition.

The present invention also provides an oral composition for prophylaxis or therapy of an oral disease comprising a lactic acid bacterium belonging to the genus *Weissella* and xylose.

The oral disease refers to caries, periodontal disease and the like.

The present invention further provides an oral composition for prophylaxis or therapy of an oral disease comprising a lactic acid bacterium W3 belonging to the genus *Weissella* and xylose.

The present invention further provides a food or beverage comprising a lactic acid bacterium W3 belonging to the genus *Weissella* or the above oral composition. The food and beverage are not particularly limited and examples thereof include confectionery, chilled/frozen desserts, dairy products, meat, fish and vegetables and processed food thereof, refreshing beverages, alcoholic beverages, water, feed for domestic animals and pet animals and the like.

An embodiment of the present invention is hereinafter represented by way of Example which does not limit the present invention.

### Example

### <Culture method and test method>

The culture method and test method used in Example are now described in detail.

### 1. Culture of Streptococcus mutans UA159 and lactic acid bacteria

The *Streptococcus mutans* strain (herein sometimes referred to as *S. mutans* or S. m) used was *Streptococcus mutans* UA159 and was anaerobically cultured in BHI (Brain Heart Infusion, Japan BD) broth at 37°C. Lactic acid bacteria were anaerobically cultured in MRS (Japan BD) broth or a plate medium thereof further containing 1.5% agar at 37°C.

### 2. Identification of species by 16S rRNA gene sequencing

In order to amplify a DNA fragment of a region of about 500 bp upstream of the 16S rRNA gene, a template, i.e. genome DNA prepared from a lactic acid bacterium was amplified by PCR with 8UA forward primer (3'-AGAGTTTGATCCTGGCTCAG-5' (SEQ ID NO: 1)) and 519B reverse primer (3'-ATTACCGC(C/G)GCTGCTG-5' (SEQ ID NO: 2)) (NPL 2). The temperature condition of the reaction was incubation at 95°C for 5 minutes followed by 25 cycles of incubation at 95°C for 30 seconds, 55°C for 30 seconds and 72°C for 30 seconds and a final incubation at 72°C for 7 minutes. PCR products were subjected to direct sequencing with 8UA forward primer. The resulting base sequences of DNA were subjected to homology search for base sequences against a gene database (DDBJ: DNA Data Bank of Japan) to try to identify the species.

### 3. Formation of S. mutans biofilms and evaluation of activity of S. mutans biofilm formation suppression

TSB (Tripticase soy broth, Japan BD) containing 0.25%-2% sucrose was added to a 96-well microplate and 20 µl of culture medium of *S. mutans* which was pre-cultured in BHI broth and diluted to have OD₆₆₀ of about 0.3 was further inoculated (the total volume of the culture medium was 300 µl). The bacterium was anaerobically cultured at 37°C for 16 hours and a culture supernatant was thereafter discarded. Biofilms formed at the bottom of the microplate were gently washed twice with saline. The biofilms were then stained with a 0.3% crystal violet solution for 10 minutes and thereafter gently washed twice with saline. The biofilms were left to dry followed by measurement of OD₅₇₀ on a microplate reader (SH-1000 Lab, Corona Electric Co., Ltd.) and the result was regarded as the biofilm index. When *S. mutans* biofilm formation suppression by lactic acid bacteria was evaluated, lactic acid bacteria were added to the culture medium so as to be 10⁷-10⁸ cells/ml. In order to examine the effect of xylose, xylose was appropriately added to the culture medium in some measurements.

### 4. Evaluation of carbohydrate assimilation of lactic acid bacteria

Carbohydrate assimilation of lactic acid bacteria was evaluated with API50 CHL kit (bioMerieux, France). In brief, a colony formed by culturing a bacterium on an MRS agar medium was suspended in a medium for seed culture (API 50 CHB/CHE medium) and prepared to have a turbidity of McFarland standard 2. The suspension was inoculated to wells for determination of metabolism of 49 different carbohydrates, overlaid with mineral oil and cultured for 24 hours. The metabolism was determined to be positive or negative by colour change of an indicator.

### 5. Evaluation of carbohydrate assimilation of S. mutans

A culture medium (50 µl) of *S. mutans* cultured in BHI broth until OD₆₆₀ of about 0.5 was obtained was inoculated to 5 ml of HI broth containing 5% of glucose, sucrose, xylose, xylooligosaccharides or fructooligosaccharides and the bacterium was anaerobically cultured at 37°C for 18 hours. The pH of the culture medium was measured and evaluated whether the measured pH was above 5.7 or not, which is the pH at which calcium and phosphoric acid begin to dissolve from teeth (enamel).

### 6. Evaluation of growth curve of lactic acid bacteria

A lactic acid bacterium was anaerobically cultured at 37°C in MRS broth until OD₆₆₀ of about 0.5 was obtained, and 50 µl of the culture medium was inoculated to 5 ml of Heart Infusion broth (HI broth, Japan BD) devoid of any carbohydrate. The bacterium was anaerobically cultured at 37°C and OD₆₆₀ was measured every hour. In order to evaluate assimilation of sucrose, xylose, xylooligosaccharides or fructooligosaccharides, the same procedure was carried out except that 1.5% of each carbohydrate was added to the HI broth.

### <Experiments and results>

Specific procedures of experiments and results carried out in the present Example are described hereinbelow.

### 1. Search for lactic acid bacteria having S. mutans biofilm formation suppressing effect

The inventors of the present application first of all studied lactic acid bacteria from kimchi and confirmed that lactic acid bacteria belonging to the genus *Weissella* strongly suppress *S. mutans* biofilm formation. This result was already demonstrated in the report by M. S. Kang et al. (NPL 3) or in PTL 1 and thus corresponded to the results reported therein.
A goal sought to be attained in the present invention was to obtain, among lactic acid bacteria belonging to the genus *Weissella,* a lactic acid bacterium belonging to the genus *Weissella* having particularly strong biofilm formation suppressing effect. Thus the inventors of the present application again spread some kimchi on MRS media and cultured to isolate a plurality of novel lactic acid bacteria strains. The lactic acid bacteria strains were Gram-stained and analysed under a microscope for morphology. A control, *Weissella cibaria* JCM12495 (reference strain), was used for microscopy and strains that were assumed to belong to the genus *Weissella* were selected. The DNA fragments of the region about 500 bp upstream of the 16S rRNA gene of the strains were further sequenced, subjected to homology search of base sequences against a gene database (DDBJ: DNA Data Bank of Japan) to select the strains that were identified to be lactic acid bacteria strains belonging to the genus *Weissella.* Ultimately 20 strains of lactic acid bacteria belonging to the genus *Weissella* were obtained. These strains were designated as W1 to W20.

### 2. Comparison of the activity of biofilm formation suppression between lactic acid bacteria belonging to the genus Weissella

The biofilm formation suppressing effect of lactic acid bacteria W1 to W20 strains belonging to the genus *Weissella* was evaluated. Controls for comparison used were known lactic acid bacteria for prophylaxis of dental decay, *Lactobacillus reuteri* and *Lactobacillus salivarius.* The results are shown in Fig. 1. It was found that the lactic acid bacterium W3 belonging to the genus *Weissella* had the strongest biofilm formation suppressing effect.

### 3. Comparison of carbohydrate assimilation between known lactic acid bacteria belonging to the genus Weissella and lactic acid bacterium W3 belonging to the genus Weissella

The carbohydrate assimilation of the lactic acid bacterium W3 belonging to the genus *Weissella* was studied. The results are shown in Table 1.

### [Table 1]

**Table 1**

| **Substrate** | **Result** | **Substrate** | **Result** |
|---|---|---|---|
| L-arabinose | - | Aesculin | **+** |
| Glycerol | - | Salicin | **+** |
| Erythritol | - | D-Cellobiose | **+** |
| D-arabinose | - | D-Maltose | **+** |
| L-arabinose | - | D-Lactose | - |
| D-Ribose | - | D-Melibiose | - |
| D-Xylose | **+** | D-Sucrose | **+** |
| L-Xylose | - | D-Trehalose | - |
| D-Adonitol | - | Inulin | - |
| β-Methyl-D-xyloside | - | D-Melezitose | - |
| D-Galactose | **+** | D-Raffinose | - |
| D-Glucose | **+** | Starch | - |
| D-Fructose | **+** | Glycogen | - |
| D-Mannose | **+** | Xylitol | - |
| L-Sorbose | - | Gentiobiose | - |
| L-Rhamnose | - | D-Turanose | - |
| Dulcitol | - | D-Lyxose | - |
| Inositol | - | D-Tagatose | - |
| D-Mannitol | - | D-Fucose | - |
| D-Sorbitol | - | L-Fucose | - |
| α-Methyl-D-mannoside | - | D-Arabitol | - |
| α-Methyl-D-glucoside | - | L-Arabitol | - |
| N-Acetyl-glucosamine | **+** | Gluconate | **+** |
| Amygdalin | - | 2-Keto-gluconate | - |
| Arbutin | **+** | 5-Keto-gluconate | - |

Assimilation of the lactic acid bacterium W3 belonging to the genus *Weissella* was different from that of reference strains, i.e. *W. confusa* and *W. cibaria.* The assimilation was also different from that of the lactic acid bacterium belonging to the genus *Weissella* disclosed in PTL 1. Therefore, the results suggested that W3 is a novel strain that is different from known lactic acid bacteria belonging to the genus *Weissella* (Table 2).

### [Table 2]

**Table 2 Comparison of carbohydrate assimilation between lactic acid bacteria belonging to the genus Weissella**

| Carbohydrate metabolism | *Weissella* W3 | W.cibaria LGM17699^{T} (reference strain) | W.confusa JCM1093^{T} (reference strain) | W.cibaria CMU,CMS-1, CMS-2,CMS-3 |
|---|---|---|---|---|
| arabinose | - | **+** | - | **+** |
| Cellobiose | **+** | **+** | **+** | **+** |
| Galactose | **+** | - | **+** | **+** |
| Lactose | - | - | - | - |
| Melibiose | - | - | - | - |
| Raffinose | - | - | - | - |
| Ribose | - | - | **+** | - |
| Salicin | **+** | **+** | **+** | **+** |
| Sucrose | **+** | **+** | **+** | **+** |
| Trehalose | - | - | - | - |
| Xylose | **+** | **+** | **+** | **+** |

### 4. Analysis of the 16S rRNA gene of the lactic acid bacterium W3 belonging to the genus Weissella

The analysis result of the base sequence of (the region about 500 bp upstream of) the 16S rRNA of the lactic acid bacterium W3 belonging to the genus *Weissella* is as follows:

From the result of the database search, the lactic acid bacterium W3 belonging to the genus *Weissella* was presumed to be *Weissella confusa* from the above sequence. However, the carbohydrate assimilation of the lactic acid bacterium W3 belonging to the genus *Weissella* is different from that of the reference strain of *Weissella confusa.*

In NPL 4, the authors indicate that it is difficult to identify the species of lactic acid bacteria belonging to the genus *Weissella* from genetic information of 16S rRNA as the base sequence of the 16S rRNA gene of *Weissella confusa* has 89.2%-99.2% homology to that of *Weissella cibaria.* By comprehensively interpreting the above, it was concluded that the lactic acid bacterium W3 belonging to the genus *Weissella* is a novel strain that is different from known strains and the bacterium was deposited to National Institute of Technology and Evaluation Patent Microorganisms Depositary (#122 2-5-8 Kazusakamatari, Kisarazu-shi, Chiba 292-0818, Japan) on 21 January 2014 as *Weissella sp.* (w3). The accession number thereof is NITE BP-01789.

### 5. Search for carbohydrates suitable to be combined with lactic acid bacteria belonging to the genus Weissella in the oral cavity

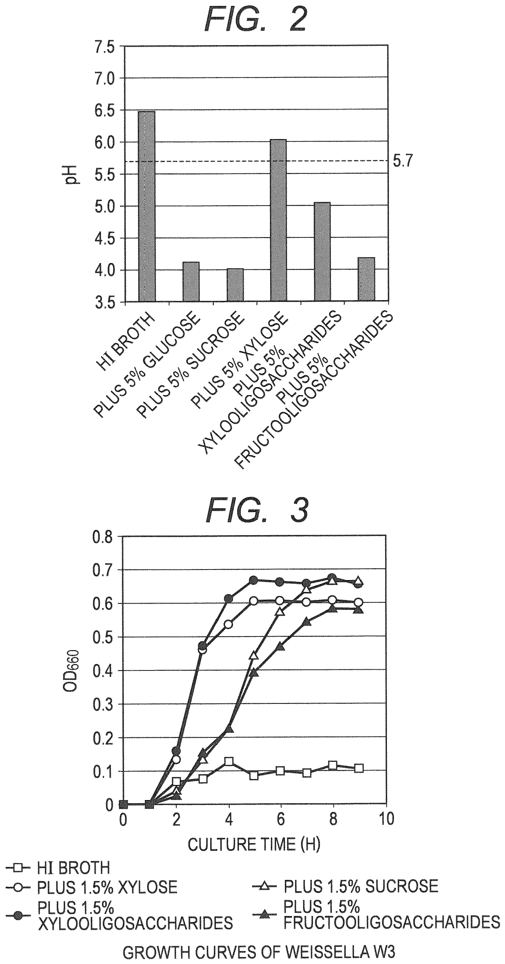

Given that lactic acid bacteria are used for prophylaxis or therapy of oral diseases, the carbohydrate that serves as a nutrient for the lactic acid bacteria is preferably the one that is less likely metabolised by general bacteria, particularly *S. mutans,* the cause of caries, and proliferates and activates solely lactic acid bacteria. In the light of the above, we focused on xylose.
As a result of the evaluation of carbohydrate assimilation of *S. mutans* (Fig. 2), it was found that the culture medium of *S. mutans* exceeded pH 5.7 even when the medium contained 5% xylose. The result indicates that *S. mutans* less likely metabolises xylose and xylose is less likely to cause dental decay. Meanwhile, the lactic acid bacterium W3 belonging to the genus *Weissella* was cultured on the limited number of carbohydrates in the media and the growth curves thereof were evaluated. As a result, as shown in Fig. 3, all of xylose, sucrose, xylooligosaccharides and fructooligosaccharides enhanced the growth of the lactic acid bacterium W3 belonging to the genus *Weissella* relative to HI broth. Among others, xylose and xylooligosaccharides strongly enhanced the growth. It is assumed that the lactic acid bacterium W3 belonging to the genus *Weissella* easily metabolises these carbohydrates.
From the above results, it is believed that xylose is a useful carbohydrate to be combined with lactic acid bacteria belonging to the genus *Weissella* in order to be used for prophylaxis or therapy of oral diseases.

### 6. Sucrose concentration dependency of the S. mutans biofilm formation suppressing effect by the lactic acid bacterium W3 belonging to the genus Weissella

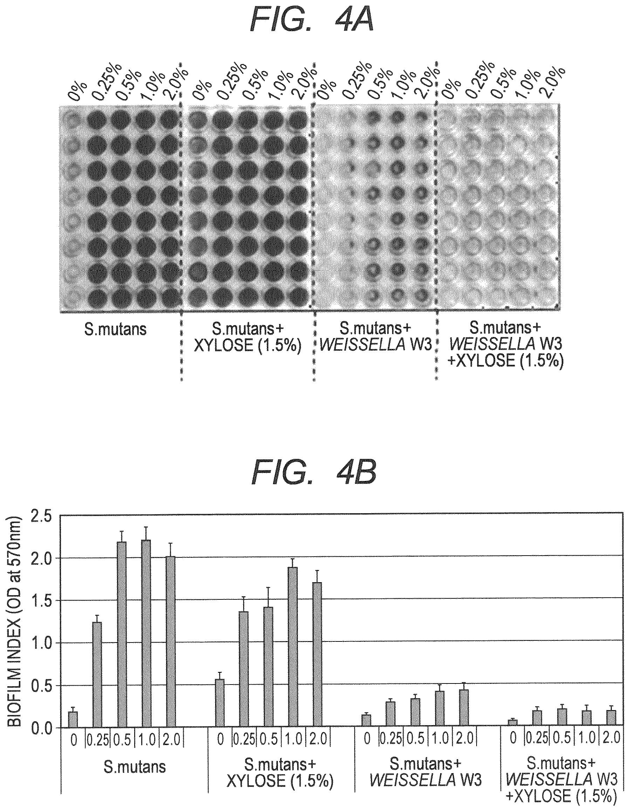

The results of evaluation of the activity of *S. mutans* biofilm formation suppression showed that the lactic acid bacterium W3 belonging to the genus *Weissella* suppressed the biofilm formation under any condition of 0.25%-2% sucrose-containing media (Figs. 4A and 4B, *S. mutans* + lactic acid bacterium W3 belonging to the genus *Weissella*). Further, the activity of *S. mutans* biofilm formation suppression was evaluated in the presence of 1.5% xylose. In the presence of high concentrations such as 1-2% of sucrose, addition of 1.5% xylose to the lactic acid bacterium W3 belonging to the genus *Weissella* significantly enhanced the biofilm formation suppressing effect compared to the case where only lactic acid bacterium W3 belonging to the genus *Weissella* was added (p < 0.05) (Figs. 4A and 4B, *S. mutans* + lactic acid bacterium W3 belonging to the genus *Weissella* + xylose).

### 7. Xylose concentration dependency of the biofilm formation suppressing effect by the lactic acid bacterium W3 belonging to the genus Weissella

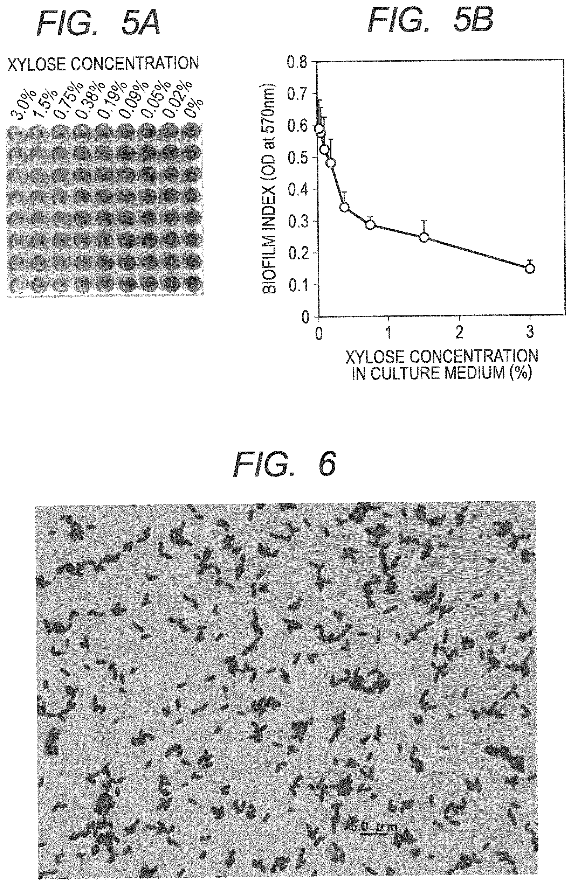

*S. mutans* was cultured in TSB containing 1% sucrose by adding the lactic acid bacterium W3 belonging to the genus *Weissella* and allowed to form biofilms. When 0.02%-3.0% xylose was added during culture, biofilm formation was suppressed in a xylose concentration dependent manner (Figs. 5A and 5B). When the result was statistically analysed by Dunnett's multiple comparison test, it was found that the broth containing 0.19% or more xylose significantly decreased biofilms compared to the broth without xylose (p < 0.05).

### 8. Microscopic analysis of the lactic acid bacterium W3 belonging to the genus Weissella

The result of morphological analysis of the Gram-stained lactic acid bacterium W3 belonging to the genus *Weissella* is shown in Fig. 6.

According to the above Example, it was found that the lactic acid bacterium W3 belonging to the genus *Weissella* suppresses *S. mutans* biofilm formation and is effective for prophylaxis or therapy of oral diseases. Moreover, the lactic acid bacterium belonging to the genus *Weissella* had an increased suppression activity when the bacterium was combined with xylose. As *S. mutans* less likely metabolises xylose, it was found that xylose is effective for prophylaxis or therapy of oral diseases when xylose is combined with a lactic acid bacterium belonging to the genus *Weissella,* particularly the lactic acid bacterium W3 belonging to the genus *Weissella.*

## Claims

1. A lactic acid bacterium W3 belonging to the genus *Weissella (Weissella sp.* (W3): accession number: NITE BP-01789) or a mutant thereof, or a transformant thereof.

2. An oral composition for prophylaxis or therapy of an oral disease comprising a lactic acid bacterium belonging to the genus *Weissella* which is selected from *Weissella* W3 (*Weissella sp.* (W3): accession number: NITE BP-01789), *W. cibaria, W. confusa,* a mutant thereof and a transformant thereof, and xylose.

3. A food or beverage comprising a lactic acid bacterium W3 belonging to the genus *Weissella* (*Weissella sp.* (W3): accession number: NITE BP-01789) or a mutant thereof, or a transformant thereof.

4. A food or beverage comprising the oral composition according to claim 2.

## Patentansprüche

1. Milchsäurebakterium W3 der Gattung *Weissella* (*Weissella sp.* (W3):
Hinterlegungsnummer: NITE BP-01789) oder ein Mutant davon, oder ein Transformant davon.

2. Oralzusammensetzung zur Prophylaxe oder Therapie einer oralen Erkrankung, umfassend ein Milchsäurebakterium der Gattung *Weissella,* das ausgewählt ist aus *Weissella* W3 (*Weissella sp.* (W3):
Hinterlegungsnummer: NITE BP-01789), *W. cibaria, W. confusa,* einem Mutant davon und einem Transformant davon, sowie Xylose.

3. Lebensmittel oder Getränk, umfassend ein Milchsäurebakterium W3 der Gattung *Weissella* W3 (*Weissella sp.* (W3): Hinterlegungsnummer: NITE BP-01789) oder ein Mutant davon oder ein Transformant davon.

4. Lebensmittel oder Getränk, umfassend die Oralzusammensetzung nach Anspruch 2.

## Revendications

1. Bactérie lactique W3 appartenant au genre *Weissella* (*Weissella sp.* (W3) : numéro d'accès : NITE BP-01789) ou une mutante de celle-ci, ou une transformation de celle-ci.

2. Composition orale pour la prophylaxie ou le traitement d'une maladie orale comprenant une bactérie lactique appartenant au genre *Weissella* qui est choisi parmi *Weissella* W3 (*Weissella sp.* (W3) : numéro d'accès : NITE BP-01789), *W. cibaria, W. confusa,* une mutante de celle-ci ou une transformation de celle-ci, et du xylose.

3. Aliment ou boisson comprenant une bactérie lactique W3 appartenant au genre *Weissella* (*Weissella sp.* (W3) : numéro d'accès : NITE BP-01789) ou une mutante de celle-ci, ou une transformation de celle-ci.

4. Aliment ou boisson comprenant la composition oral selon la revendication 2.
